(19) Europäisches Patentamt
European Patent Office
Office européen des brevets

(11) **EP 1 639 956 B1**

(12) **EUROPEAN PATENT SPECIFICATION**

(45) Date of publication and mention
of the grant of the patent:
**18.07.2007 Bulletin 2007/29**

(51) Int Cl.:
***A61B 18/12*** *(2006.01)*

(21) Application number: **04022985.8**

(22) Date of filing: **27.09.2004**

(54) **Arrangement for therapy of tumours**

Anordnung zur therapeutischen Behandlung von Tumoren

Dispositif de thérapie de tumeurs

(84) Designated Contracting States:
**AT BE BG CH CY CZ DE DK EE ES FI FR GB GR HU IE IT LI LU MC NL PL PT RO SE SI SK TR**

(43) Date of publication of application:
**29.03.2006 Bulletin 2006/13**

(73) Proprietor: **VibraTech AB**
**100 54 Stockholm (SE)**

(72) Inventors:
• **Wiksell, Hans**
**187 51 Täby (SE)**
• **Auer, Gert**
**171 60 Solna (SE)**

• **Ekstrand, Vilhelm**
**112 27 Stockholm (SE)**
• **Harge, Peter**
**133 33 Saltsjöbaden (SE)**

(74) Representative: **Holmberg, Nils Anders Patrik et al**
**Dr Ludwig Brann Patentbyrå AB**
**P.O. Box 17192**
**104 62 Stockholm (SE)**

(56) References cited:
**US-A- 6 066 139**         **US-A1- 2001 056 280**
**US-A1- 2002 052 600**     **US-A1- 2004 092 926**
**US-B1- 6 190 381**

**Description**

**Technical field**

The present invention relates to an arrangement for the therapy of tumours, which is applicable to the Radio Frequency Ablation (RFA) technique. In comparison with prior art RFA arrangements, the arrangement according to the present invention provides for a more effective treatment of the tumour. In a preferred embodiment of the present invention, the arrangement comprises temperature control means that provides for a more accurate temperature control. In another preferred embodiment said arrangement provides for a better penetration of the needle into the tumour in comparison with RFA arrangements in the prior art.

**Background art**

Many women in the western world faces the prospect of breast cancer. The tendency in modern breast cancer treatment has been towards less invasive local treatment of the tumour and it is also becoming more important in modern therapy to minimise the alternation of the breast configuration due to treatment. Breast conservation surgery has thus in many cases gained in favour over radical mastectomy. The goal of radical surgery is to remove all of the malignant breast tissue, often combined with lymph node resection, resulting in a considerable hospitalisation period and later need of breast restitution. Furthermore, a growth zone is resected to prevent local reoccurrence. With modern diagnostic methods, as routine mammography screening, many small tumours are detected. In cases where the tumour is still not spread (cancer in situ), conventional breast surgery might be seen as an "overkill" procedure. Thus, minimally invasive approaches other than traditional surgery have been explored, with which other potential benefits often are obtained, such as reduced morbidity rates, reduced treatment duration, ability to perform therapy even when patients are in bad medical condition and possibility to keep the procedure on an outpatient basis.

One such approach is the Radio Frequency Ablation (RFA) technique, which causes thermal destruction of the tumour by coagulation necrosis. Liver tumours are the most common type of tumours that are treated with RFA but the treatment is now being performed in other areas of the body, such as the lungs, bones, prostate and adrenal glands. The use of RFA in breast cancer treatment is relatively new but studies performed by the inventors have shown that the technique is well suited for treatment of breast tumours and especially ductal breast tumours.

The RFA monopolar technique involves inserting a needle directly into the tumour to be treated and applying a larger planar ground electrode to an outer surface of the body. When radio frequency (RF) energy, generated by a power source, is applied between the ground electrode and the needle, the latter will act as a counter electrode and a current path is established between the electrodes. The current density at the needle will be much smaller than at the ground electrode, thus the latter is often referred to as the indifferent electrode and associated with no temperature elevation. Temperature elevation in the tissue is caused by ion agitation, which is converted by means of friction into heat, thus the RF energy heats the tissue by way of the tissue's electric resistance. The heat, that is generated in the tissue surrounding the needle, destroys the tumour cells, without nerve stimulation and pain. The denaturised cells are left in-situ and are later resorbed by the body. In order to avoid regrowth of the tumour, an amount of surrounding tissue, growth zone, often needs to be destroyed as well. The total volume affected by the excess temperature is known as the lesion volume and thus the shape and size of the affected area is known as the lesion shape and lesion size, respectively.

It is well known that tissue exposed to excess temperature during a sufficient long time is completely killed. In a technique generally referred to as hyperthermia, the temperature generated is approximately 42-43°C. With an excess temperature in this range, the exposure time needed to effectively treat a tumour, is normally a number of minutes, sometimes up to one hour, depending on for instance the lesion size. Thus, hyperthermia temperatures within the field of RFA inevitably prolongs the treatment time of the patient, brings about unnecessary suffering and often produces nonuniform lesioning of the tumour.

The tissue heating is constantly cooled during treatment, both passively through thermal conduction as well as actively through blood perfusion. Thus, the latter contributes to a large extent to remove heat. However, blood perfusion is affected by heat. Moderate increases in temperature also increase blood perfusion, but at higher excess temperatures, the blood perfusion collapses. Thus the latter results in that the temperature diffusion increases enormously.

Moreover, it is important that the temperature do not exceed -100°C, i.e. the boiling point for water. If so happens, gas bubbles are created at the needle surface which tend to isolate the electrode from the tissue. Also, at sufficiently high temperatures, tissue adjacent to the electrode surface will get charred and thus impair further heating of the tissue, due to the drastic decrease in conductivity. Thus, the size of the lesion volume is limited because of the above mentioned effects.

Therefore, in order to obtain an effective treatment of the tumour, it is of importance that the temperature is monitored and controlled effectively and that the needle is cooled in an effective way .

The output-power of the RF source regulates the generated temperature in the tissue. Within the RFA technique, it is known to measure for instance the impedance in the circuit comprising the needle and the indifferent ground electrode as well as measuring the temperature at the needle-tip during treatment and adjusting the voltage between the electrodes according to the measured values of the impedance and the temperature. It is also known to continuously feed the values of the impedance and the temperature during treatment to a control unit, which will send feed-back signals to the RF-source, increasing or decreasing the output-power of said source.

An increase in the impedance indicate that bubbles have been created or that tissue charring have occurred. In order to cool the needle, to prevent tissue charring and bubble formation, it is known to use needles which utilize cooling media in the interior of the needle. One of the results of having such cooling media inside the needle, is that the true maximum temperature of the tissue temperature, due to temperature deviation, is not located at the needle surface but at a certain distance from said needle inside the tissue. This makes it difficult to measure the true maximum temperature of the tissue and the known means for temperature control of the tissue temperature have not proven to be satisfying enough.

In the recent years, it has also been possible to detect smaller and smaller tumours, for instance through the technical development of screening mammography and ultrasound diagnostic scanning. It is not unusual to detect tumours which are in the size of just a few millimetres. Especially these small tumours but also tumours of sizes 10-20 mm in diameter are frequently non-palpable and undergo anatomical distortion when hit by the needle. The reason is that the glandular breast tissue is surrounded by a loose fatty and connective tissue, which makes it difficult to correctly position and move the needle into the tumour and the problem aggravates if the tumour is hard or fibrous, which is most often the case. Thus, the insertion of needles into the tumour is often difficult or even impossible.

At the present the RFA technique is, when it is applied in cancer treatment, most often viewed as palliative, but not curative and the technique is in most cases used to shrink tumours so that mastectomy can be avoided. Thus, there is a need for improvements of the RFA technique, such as a shortening of the actual treatment time and at the meantime rendering the killing of the tumour cells more effective. Also, there is a need for a more accurate temperature control of the tissue temperature. Furthermore, there is a need for an RFA arrangement that provides for a more effective penetration of the tumour to be treated, especially small and hard fibrous tumours.

An RFA arrangement according to the preamble of claim 1 is disclosed in US 2004/0092926.

### Disclosure of the Invention

The arrangement according to the invention provides means for a more effective penetration of the tumour to be treated in comparison with prior art RFA arrangements, as defined in claim 1.

In a preferred embodiment, the arrangement according to the present invention provides means for a more accurate temperature control of the tissue temperature during treatment in comparison with prior art RFA arrangements.

Further preferred embodiments are set forth in the dependent claims.

### Brief Description of Drawings

Fig. 1 schematically shows the inventive arrangement with a first set of temperature measurement means,
Fig. 2 schematically shows the inventive arrangement with a second set of temperature measurement means,
Fig. 3 schematically shows a cross section of the needle of the inventive arrangement,
Fig. 4 shows the flow dependent measured temperatures at different calibration temperatures.

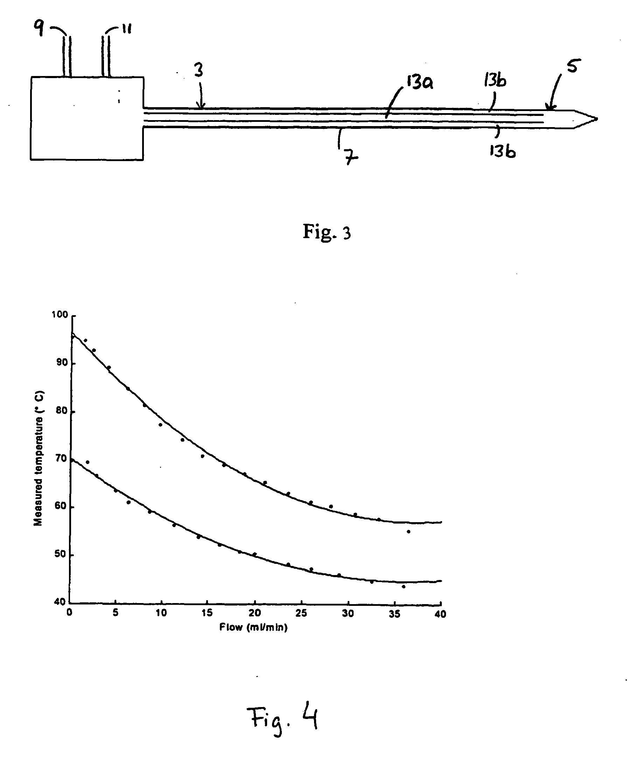

The inventive arrangement comprises a needle 2 (Fig. 3) and a planar ground electrode 4, that is to be applied to an outer surface of the patient's body, preferably the back of the patient. The dimensions of the needle 2 depend on for instance the size and shape of the tumour 1 to be treated. Preferably the needle 2 has an active part of a length of approximately 50-100 mm and a diameter in the range of 1-4 mm. The arrangement further comprises a low impedance RF generating device 10, that is adapted to apply RF energy between the ground electrode 4 and the active part of the needle 2, when the needle 2 is correctly positioned inside the tumour 1 to be treated. When RF energy is applied, the needle 2 acts as a counter electrode, such that a current path is established between the electrodes 2, 4, generating heat in a spherical lesion 12 of tissue surrounding the needle 2, as described above.

In one embodiment, the tissue temperature is in the range of 50-99°C, preferably 70-95°C , i.e. the so called thermotherapy temperature, and thus considerably higher than the commonly used hyperthermia temperature. Due to the dissimilar physical parameters of tumour and adipose (fat) tissue, the heat "is drawn" to the tumour. The tumour has a higher electric conductivity than the surrounding adipose tissue. As a consequence, the tumour attracts the electric energy from the fat. Thus, the specific absorption rate (SAR) is relatively higher in the tumour than in the adipose tissue. In fact, the inventors have observed that thin tumour strips extending from the core tumour are killed but the surrounding fat are however unaffected.

In order to achieve an effective treatment, the time needed to maintain the elevated temperature, is dependent on the lesion size and shape. Normally this time is in the range of 5-15 minutes, thus a shorter treatment time than would be needed with a temperature within the hyperthermia temperature range, in the treatment of the same tumour(s). The output power of the RF generating device regulates the elevated tissue temperature. Generally, the RF generating device operates in the range of 5-100 W, preferably 30 W. The applied RF frequency may be in the range of 0,2-20 MHz but is preferably approximately 1,5M Hz. According to the invention the power of the RF generating device is automatically adjusted during the treatment as described below.

With reference to figure 3, the needle 2 of the inventive arrangement is provided with an insulated part 3 and a non-insulated part 5, by covering the exterior of the needle with an insulating material 7, for instance Teflon®.

According to a preferred embodiment of the inventive arrangement, the needle 2 that is to be inserted to the tumour 1 is further provided with means that enables circulation of a cooling liquid inside the needle 2. The cooling liquid is guided into the needle through a liquid inlet 9 and along a liquid inflow channel 13a, provided in the middle of the needle 2 along the horizontal axis of said needle 2. At the needle tip the cooling liquid changes flow direction and is guided back through a liquid outflow channel 13b, i.e. the empty space between the liquid inflow channel 13a and the outer surface of the needle, and out through a liquid oulet 11. Liquid inlet 9 and liquid outlet 11 are preferably provided with Luer-Lock connections (not shown). The means that enables circulation of a cooling liquid inside the needle 2, further comprises a liquid flow generating device 14, for instance a pump or the like, which is adapted to direct the liquid into the needle 2 through liquid inlet 9, through inflow and outflow channels 13a,b and out through liquid outlet 11, preferably with a constant, pre-determined flow rate. The diameter of the channels 13a,b is thus preferably sufficient to permit an equal flow in the liquid inflow and outflow channels. The preferred flow rate is in the range of 5-30 ml/min. The cooling liquid is preferably a sterile isotone liquid in order to protect against possible leakage. The cooling liquid is preferably water but other liquids may be used as well, such as for instance saline solution or ethanol.

The needle 2 is preferably provided as a disposable article. Alternatively, the needle 2 is adapted to be sterilized, preferably by the use of autoclavation. However other conventionally used sterilization methods may be used as well.

According to a preferred embodiment the arrangement is further provided with temperature control means. Said means comprises means (not shown) adapted to measure the flow value, Q, of the liquid circulating inside the needle 2, preferably provided in the liquid flow generating device 14. Said means is for instance a generally known flow measuring sensor or the like.

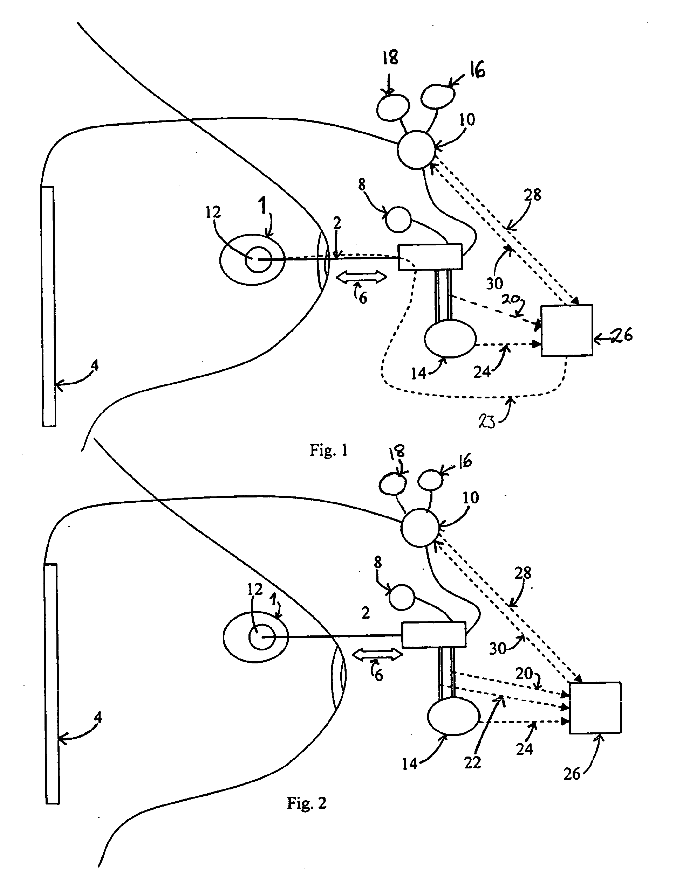

The temperature control means further comprises means adapted to measure the temperature of the needle 2 during treatment. Said temperature measurement means may comprise (a) generally known thermosensor(s) or optical fibres (not shown) incorporated in the non-insulated part of the needle 2 and a thermosensor to measure the temperature of the inlet liquid, $T_1$, preferably provided in the liquid inlet 9 (first set of temperature measurement means, fig.1). In this instance, the temperature of the needle, $T_{meas}$, and $T_1$, as measured by the first set of temperature measurement means as well as the value of the liquid flow, $Q=dV/dt$ (ml/s), is continuously fed 23, 20, 24 to a control unit 26, preferably a computer or the like. The control unit 26 is provided with means that according to the value of $T_{meas}$, $T_1$ and the value of the flow Q, will calculate the true temperature of the tissue, $T_{tissue}$, as explained in further detail below.

Alternatively, the temperature measurement means of the preferred embodiment may comprise means (not shown) for measurement of the temperature value of the inlet liquid, $T_1$, as well the temperature value of the outlet liquid, $T_2$ (second set of temperature measurement means, fig.2). Said means is preferably generally known thermosensors, provided for instance in the liquid inlet 9 and the liquid outlet 11, respectively. In the alternative embodiment of the preferred embodiment (Fig. 2), $T_1$, $T_2$ and the value of the liquid flow, $Q=dV/dt$ (ml/s), is continuously fed 20, 22, 24 to the control unit 26. In this instance, the control unit 26 is provided with means that according to the value of the temperature difference between $T_1$ and $T_2$, i.e. $T_{dff}=T_2-T_1$, and the value of the flow Q, will calculate the true temperature of the tissue, $T_{tissue}$.

According to the preferred embodiment of the inventive arrangement, the control unit 26 is also continuously fed 28 with values of the impedance, Z, in the circuit comprising the needle 2 and the indifferent ground electrode 4, as well as the output power, $P=U*I$, of the RF generating device 10 that regulates the generated elevated tissue temperature. Thus, the inventive arrangement comprises generally known means 16 adapted to measure the impedance and generally known means 18 adapted to measure the output power. The control unit 26 is accordingly further provided with means that depending on the values of Z, P and the true tissue temperature, $T_{tissue}$, will send feed-back signals 30 to the RF generating device 10, increasing or decreasing the output-power of said RF generating device, depending on if $T_{tissue}$ is to be increased or decreased, respectively.

The power needed to maintain a certain tissue temperature, also referred to as the heat resistance, is a good indicator of the perfusion status of the tissue, i.e. the degree of tissue destruction, and the perfusion collapse might be a good indicator on the quality of the therapy session. Furthermore the measured impedance between the treatment and the indifferent electrodes is a good indicator of the status around the electrode, i.e. if charring and/or bubbles is/are present. Thus the control unit 26 is preferably provided with means to calculate the heat resistance as a tool to monitor the tissue perfusion.

The use of the value of Q when determining the true tissue temperature value, $T_{tissue}$, as well as using said values for sending feed-back signals to the RF generating device provides for a more accurate temperature control of the tissue temperature in comparison with prior art RFA arrangements.

The inventive arrangement functions in combination with either of above mentioned means for temperature measurement. The use of the first set of temperature measurement means, i.e. for instance thermosensor(s) incorporated in the needle is a straight forward temperature measurement method. The measurement of $T_1$ and $T_2$, respectively, may however provide for a less expensive temperature measurement than the use of thermosensor(s) in the needle, since if the dimensions of the needle are decreased, the incorporation of thermosensors in the needle may be problematic and a rather expensive procedure.

The low level signal obtained from the thermosensors may suffer from interference from the applied RF-power field. In this instance the arrangement is preferably provided with low-pass filtering means (not shown) in order to filter the RF-signal to obtain high quality measurements in the extreme RF-field environment. Moreover, the arrangement is preferably designed as cardiac floating (CF) to guarantee patient leakage current (PLC) and earth leakage current (ELC), according to EN 60-601-1 international standard and EC.

According to the invention, the arrangement is provided with a longitudinal movement means 8, adapted to apply an oscillating longitudinal movement 6 to the needle 2. Longitudinal oscillation 6 of the needle 2 with suitable frequency and amplitude will due to it's acceleration component effectively penetrate even small and hard tumours, for instance a tumour of just a few millimeteres, and the inventors have in fact discovered that the resistance at penetration may be reduced by as much up to ten times when a frequency of 250 Hz is applied. Depending on for instance the dimensions of the needle 2 and the size, condition and localization of the tumour 1, the operator of the arrangement, for instance the physician responsible for the treatment, is capable of, and free to, choose a suitable frequency, amplitude and wave form that is to be applied to the needle 2 when said needle is about to penetrate the tumour. Preferably the applied sinusoidal frequency is in the range of 30-300 Hz and the amplitude in the range of 0-4 mm. Penetration force is decreasing with increasing frequency and amplitude and acceleration forces increase with the parameters.

The oscillating movement 6 of the needle 2 is applied in order to provide for a better penetration of the needle into the tumour 1 and may be occluded during the insertion of the needle into the tumour 1 if the operator of the arrangement so desires.

Calculation of the true tissue temperature using the first set of temperature measurement means

The true tissue temperature $T_{tissue}$, is determined by using the values of $T_{meas}$ and the value of the flow Q, for instance according to equation (1):

$$dT = T_{tissue} - T_{meas} = k_1(T_{tissue}) * Q + k_2(T_{tissue}) * Q^2 \qquad (1)$$

wherein dT is the temperature gradient over the needle and $k_1$ and $k_2$ are constants.

$k_1$ and $k_2$ are found using calibration. For instance, fig. 4 shows the flow dependent measured temperatures, $T_{meas}$, at different calibration temperatures (serving as fictitious tissue temperatures), using a bath unit kept at temperatures 70°C (lower curve) and 95°C (upper curve). In the calibration measurements the bath unit was kept at said calibration temperatures, respectively, and $T_{meas}$ was measured at 20 different flow rates. The cooling liquid circulating inside the needle was kept at 15°C . Using least square algorithms, $k_1$ and $k_2$ were calculated for tissue temperatures 70 and 95°C. At a constant flow rate there is a linear relationship between $T_{meas}$ and $T_{tissue}$. Thus $T_{tissue}$ is calculated by linear extrapolation between the 70 and 95°C curves in all flows. If the temperature of the inlet flow is changed the constants $k_1$ and $k_2$ are also changed according to a known relation.

Calculation of the true tissue temperature using the second set of temperature measurement means

The true tissue temperature $T_{tissue}$ is in this scheme determined by the difference in inlet and outlet temperatures $T_{diff}=T_1 -T_2$ instead of $T_{meas}$. Otherwise, the procedure preferably follows the same scheme as the above described measurement calculation method.

Even though the inventive arrangement has been described by means of a procedure for treatment of a human breast tumour, the arrangement shall not be regarded as being limited to such. The arrangement is fully functional with other kinds of tumours, such as for instance prostate tumours as well as with animal patients.

It will be understood that the invention is not restricted to the above-described exemplifying embodiments thereof and that several conceivable modifications of the invention are possible within the scope of the following claims.

**Claims**

1. Arrangement for treatment of a tumour localized inside a body, comprising a needle (2) that is intended to be inserted into said tumour, a ground electrode (4) that is intended to be applied to the outer surface of the body, and a radio frequency generating device (10) that is intended to apply radio frequency energy between the needle and the ground electrode such that heat is generated in tissue surrounding the needle **characterized in that** the arrangement comprises longitudinal movement means (8) adapted to apply an oscillating longitudinal movement to the needle, preferably applied upon insertion of the needle into said tumour.

2. Arrangement according to claim 1 **characterized in that** the needle (2) is provided with a liquid inlet (9) communicating with a liquid inflow channel (13a) and a liquid outlet (11) communicating with a liquid outflow channel (13b), said channels are provided in the interior of the needle (2) along the horizontal axis of said needle; and **in that** the arrangement is provided with a liquid flow generating device (14) adapted to generate a flow of liquid in said channels (13a, 13b).

3. Arrangement according to claim 2 **characterized in that** the arrangement is provided with means adapted to measure the flow value of the liquid.

4. Arrangement according to any of claims 1-3 **characterized in that** the arrangement comprises means adapted to measure the temperature of the needle (2).

5. Arrangement according to claim 4 **characterized in that** the means adapted to measure the temperature of the needle (2) comprises thermosensor(s) incorporated in the needle (2).

6. Arrangement according to claim 4 **characterized in that** the means adapted to measure the temperature of the needle (2) comprises means adapted to measure the temperature of the liquid in the liquid inlet (9), means adapted to measure the temperature of the liquid in the liquid outlet (11) and means adapted to measure the flow value of the liquid.

7. Arrangement according to any of claims 1-6 **characterized in that** the arrangement comprises means (16) for measuring the impedance in the circuit comprising the needle (2) and the ground electrode (4) and means (18) adapted to measure the output power of the radio frequency generating device (10).

8. Arrangement according to any of claims 1-7 **characterized in that** the arrangement comprises a control unit (26), said control unit is:

   adapted to receive values of:

   a) the liquid in the channels (13a,13b) of needle (2) and
   b) the temperature of the needle (2) and/or the temperature of the liquid in the liquid inlet (9) and the temperature of the liquid in the liquid outlet (11);
   c) the impedance in the circuit comprising the needle (2) and the ground electrode (4),
   d) the output power of said radio frequency generating device (10)

   provided with meant adapted to calculate the tissue temperature depending on the values of a) and b), and adapted to send signals (30) to the radio frequency generating device (10) with instructions to decrease or increase the output power of said radio frequency generating device depending on if the tissue temperature is intended to be decreased or increased.

9. Arrangement according to claim 8 **characterized in that** the control-unit (26) is provided with means to calculate the power needed of the radio frequency generating device (10) in order to maintain a predetermined tissue temperature.

10. Arrangement according to claim 9 **characterized in that** the predetermined tissue temperature is in the range of 50-99°C.

11. Arrangement according to claim 10 **characterized in that** the predetermined tissue temperature is in the range of 70-95°C.

**12.** Arrangement according to any of claims 1-11 **characterized in that** the tumour is a breast tumour.

**Patentansprüche**

**1.** Anordnung zur Behandlung eines Tumors, welcher sich in einem Körper befindet, enthaltend eine Nadel (2), welche dazu bestimmt ist, in den genannten Tumor eingeführt zu werden, eine Erdelektrode (4), welche dazu bestimmt ist, an die äußere Oberfläche des Körpers angelegt zu werden, und eine Radiofrequenz erzeugende Vorrichtung (10), welche dazu bestimmt ist, Radiofrequenzenergie zwischen der Nadel und der Erdelektrode derart anzulegen, dass Wärme in Gewebe, welche die Nadel umgibt, erzeugt wird, **dadurch gekennzeichnet, dass** die Anordnung Längsbewegungsmittel (8) aufweist, welche geeignet sind, eine oszillierende Längsbewegung auf die Nadel zu übertragen, vorzugsweise nach dem Einführen der Nadel in den genannten Tumor.

**2.** Anordnung nach Anspruch 1, **dadurch gekennzeichnet, dass** die Nadel (2) mit einem Flüssigkeitseinlass (9) versehen ist, welcher mit einem Flüssigkeitseinströmkanal (13a) in Strömungsverbindung steht, und einem Flüssigkeitsauslass (11), welcher mit einem Flüssigkeitsausströmkanal (13b) in Strömungsverbindung steht, wobei die Kanäle im Inneren der Nadel (2) längs der Horizontalachse der genannten Nadel vorgesehen sind; und dass die Anordnung mit einer Flüssigkeitsströmungserzeugungsvorrichtung (14) versehen ist, welche dazu angepasst ist, eine Strömung von Flüssigkeit in den genannten Kanälen (13a, 13b) zu erzeugen.

**3.** Anordnung nach Anspruch 2, **dadurch gekennzeichnet, dass** die Anordnung mit Mitteln ausgestattet ist, welche dazu angepasst sind, den Strömungswert der Flüssigkeit zu messen.

**4.** Anordnung nach einem der Ansprüche 1 bis 3, **dadurch gekennzeichnet, dass** die Anordnung Mittel aufweist, welche dazu angepasst sind, die Temperatur der Nadel (2) zu messen.

**5.** Anordnung nach Anspruch 4, **dadurch gekennzeichnet, dass** die Mittel, welche dazu angepasst sind, die Temperatur der Nadel (2) zu messen, (einen) Thermosensor(en) aufweisen, welcher/welche in die Nadel (2) eingebaut ist/sind.

**6.** Anordnung nach Anspruch 4, **dadurch gekennzeichnet, dass** die Mittel, welche dazu angepasst sind, die Temperatur der Nadel (2) zu messen, Mittel aufweisen, welche dazu angepasst sind, die Temperatur der Flüssigkeit in dem Flüssigkeitseinlass (9) zu messen, Mittel, welche dazu angepasst sind, die Temperatur der Flüssigkeit in dem Flüssigkeitsauslass (11) zu messen, und Mittel, welche dazu angepasst sind, den Strömungswert der Flüssigkeit zu messen.

**7.** Anordnung nach einem der Ansprüche 1 bis 6, **dadurch gekennzeichnet, dass** die Anordnung Mittel (16) zum Messen der Impedanz in dem Kreis aufweist, welcher die Nadel (2) und die Erdelektrode (4) enthält, und Mittel (18), welche dazu angepasst sind, die Ausgangsleistung der Radiofrequenz erzeugenden Vorrichtung (10) zu messen.

**8.** Anordnung nach einem der Ansprüche 1 bis 7, **dadurch gekennzeichnet, dass** die Anordnung eine Kontrolleinheit (26) aufweist, wobei die genannte Kontrolleinheit dazu angepasst ist, Werte aufzunehmen von:

a) der Flüssigkeitsströmung in den Kanälen (13a, 13b) der Nadel (2) und
b) der Temperatur der Nadel (2) und/oder der Temperatur der Flüssigkeit in dem Flüssigkeitseinlass (9) und der Temperatur der Flüssigkeit in dem Flüssigkeitsauslass (11);
c) der Impedanz in dem Kreis, welcher die Nadel (2) und die Erdelektrode (4) enthält,
d) der Ausgangsleistung der genannten Radiofrequenz erzeugenden Vorrichtung (10)

versehen mit Mitteln, welche dazu angepasst sind, die Gewebetemperatur anhängig von den Werten von a) und b) zu errechnen und
dazu angepasst sind, Signale (30) an die Radiofrequenz erzeugende Vorrichtung (10) mit Instruktionen zu senden, um die Ausgangsleistung der genannten Radiofrequenz erzeugenden Vorrichtung zu senken oder zu erhöhen, abhängig davon, ob die Gewebetemperatur abgesenkt oder erhöht werden soll.

**9.** Anordnung nach Anspruch 8, **dadurch gekennzeichnet, dass** die Kontrolleinheit (26) mit Mitteln zum Errechnen der Leistung ausgestattet ist, welche für die Radiofrequenz erzeugende Vorrichtung (10) benötigt wird, um eine vorbestimmte Gewebetemperatur aufrechtzuerhalten.

**10.** Anordnung nach Anspruch 9, **dadurch gekennzeichnet, dass** die vorbestimmte Gewebetemperatur im Bereich von 50°C bis 99°C liegt.

**11.** Anordnung nach Anspruch 10, **dadurch gekennzeichnet, dass** die vorbestimmte Gewebetemperatur im Bereich von 70°C bis 95°C liegt.

**12.** Anordnung nach einem der Ansprüche 1 bis 11, **dadurch gekennzeichnet, dass** der Tumor ein Brusttumor ist.

**Revendications**

**1.** Dispositif pour le traitement d'une tumeur localisée à l'intérieur d'un corps comprenant une aiguille (2) destinée à être insérée dans ladite tumeur, une prise de terre (4) destinée à être appliquée sur la surface externe du corps et un dispositif générant des radiofréquences (10) destiné à appliquer de l'énergie radiofréquence entre l'aiguille et la prise de terre de sorte que de la chaleur est produite dans le tissu entourant l'aiguille **caractérisé en ce que** le dispositif comprend des moyens de mouvements longitudinaux (8) conçus pour l'application d'un mouvement oscillatoire longitudinal à l'aiguille, ce mouvement est de préférence appliqué pendant l'insertion de l'aiguille dans ladite tumeur.

**2.** Dispositif selon la revendication 1, **caractérisé en ce que** l'aiguille (2) est équipée d'une entrée de liquide (9) communiquant avec un passage d'arrivée de liquide (13a) et une sortie de liquide (11) communiquant avec un passage pour l'écoulement du liquide (13b), lesdits canaux sont à l'intérieur de l'aiguille (2) le long de l'axe horizontal de ladite aiguille ; et **en ce que** le dispositif est équipé d'un système engendrant l'écoulement du liquide (14) conçu pour engendrer un écoulement de liquide dans lesdits passages (13a, 13b).

**3.** Dispositif selon la revendication 2, **caractérisé en ce que** le dispositif est équipé de moyens conçus pour mesurer le débit du liquide.

**4.** Dispositif selon l'une quelconque des revendications 1 à 3 **caractérisé en ce que** le dispositif comprend des moyens conçus pour mesurer la température de l'aiguille (2).

**5.** Dispositif selon la revendication 4, **caractérisé en ce que** les moyens conçus pour mesurer la température de l'aiguille (2) comprennent un ou des détecteurs thermiques intégrés dans l'aiguille (2).

**6.** Dispositif selon la revendication 4 **caractérisé en ce que** les moyens conçus pour mesurer la température de l'aiguille (2) comprennent des moyens conçus pour mesurer la température du liquide dans l'entrée de liquide (9), des moyens conçus pour mesurer la température du liquide dans la sortie de liquide (11) et des moyens conçus pour mesurer le débit du liquide.

**7.** Dispositif selon l'une quelconque des revendications 1 à 6, **caractérisé en ce que** le dispositif comprend des moyens (16) pour mesurer l'impédance dans le circuit comprenant l'aiguille (2) et la prise de terre (4) et des moyens (18) conçus pour mesurer la puissance de sortie du dispositif générant des radiofréquences (10).

**8.** Dispositif selon l'une quelconque des revendications 1 à 7, **caractérisé en ce que** le dispositif comprend une unité de régulation (26), ladite unité de régulation est :

conçue pour réceptionner des valeurs :

a) du débit du liquide dans les passages (13a, 13b) de l'aiguille (2) et
b) de la température de l'aiguille (2) et/ou de la température du liquide dans l'entrée de liquide (9) et de la température du liquide dans la sortie de liquide (11) ;
c) de l'impédance dans le circuit comprenant l'aiguille (2) et la prise de terre (4) ;
d) de la puissance de sortie dudit dispositif générant des radiofréquences (10)

équipée avec des moyens conçus pour calculer la température du tissu en fonction des valeurs de a) et b) ; et conçue pour envoyer des signaux (30) vers le dispositif générant des radiofréquences (10) avec des instructions pour diminuer ou augmenter la puissance de sortie dudit dispositif générant des radiofréquences selon que la température du tissu doive être diminuée ou augmentée.

**9.** Dispositif selon la revendication 8, **caractérisé en ce que** l'unité de régulation (26) est équipée de moyens pour calculer la puissance nécessaire du dispositif générant des radiofréquences (10) pour maintenir une température de tissu prédéterminée.

**10.** Dispositif selon la revendication 9, **caractérisé en ce que** la température de tissu prédéterminée est dans la gamme allant de 50 à 99°C.

**11.** Dispositif selon la revendication 10, **caractérisé en ce que** la température de tissu prédéterminée est dans la gamme allant de 70 à 95°C.

**12.** Dispositif selon l'une quelconque des revendications 1 à 11, **caractérisé en ce que** la tumeur est une tumeur du sein.

Fig. 1

Fig. 2

**Fig. 3**

**Fig. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040092926 A